# EUROPEAN PATENT APPLICATION

(11) **EP 2 476 670 A1**
(43) Date of publication of application: **18.07.2012**
(21) Application number: 11000089.0
(22) Date of filing: 07.01.2011
(51) Int. Cl.: C07D 239/34, A61K 31/513

(54) **Stable solid salts of ambrisentan**

(71) Applicant: Zentiva, K.S., 102 37 Praha 10 (CZ); Sanofi-Aventis Deutschland GmbH, 65926 Frankfurt am Main (DE)
(72) Inventor: Nagel, Norbert, 64546 Mörfelden-Walldorf (DE); Baumgartner, Bruno, 64287 Darmstadt (DE); Bröckelmann, Martin, 65428 Rüsselsheim (DE); Radl, Stanislav, 250 84 Praha-Kvetnice (CZ); Klecan, Ondrej, 282 01 Cesky Brod (CZ); Ridvan, Ludek, 102 00 Praha 10 (CZ); Dammer, Ondrej, 338 44 Dobriv (CZ)
(74) Representative: Jirotkova, Ivana

(57) **Abstract**

New solid salts of ambrisentan, including the phosphate salt, ambrisentan sodium salt and ambrisentan tert-butylammonium salt, in crystalline, amorphous, anhydrous or hydrated form.

## Description

### Field of the invention

The invention relates to stable solid salts of (*S*)-2-(4,6-dimethylpyrimidin-2-yloxy)-3-methoxy-3,3-diphenylpropanoic acid (ambrisentan, formula I).

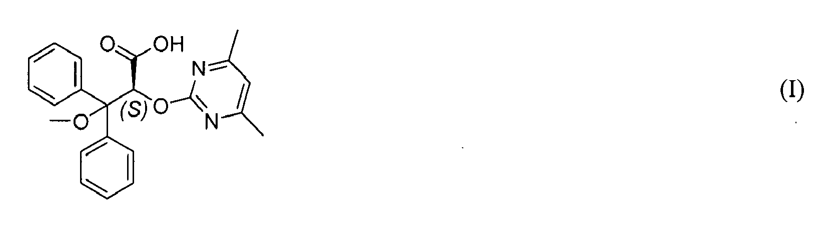

### Background of the invention and prior art

Ambrisentan (Gilead, US trade name Letairis®; EU trade name Volibris®) is a selective endothelin A receptor antagonist which is used for the treatment of pulmonary arterial hypertension (PAH). The structure of ambrisentan is revealed in W09611914 (see compound I-100, page 35). The chemical name is (6)-2-(4,6-dimethylpyrimidin-2-yloxy)-3-methoxy-3,3-diphenylpropanoic acid. The synthesis of ambrisentan is described in WO2010091877.

An amorphous form of ambrisentan is described in W02010017918. A crystalline form of ambrisentan, Form R, is described in W02010091877 and DE 202009009917. The crystalline Form R was characterized by X-ray powder diffraction (XRPD) pattern with characteristic reflections at 2-theta values of 8.9°, 12.3°, 17.9° and 26.9° ± 0.2°.

Although ambrisentan molecule contains both acidic and basic group, no salts have been described.

### Summary of the invention

The invention relates to new solid salts of ambrisentan: the phosphate salt, ambrisentan sodium salt and ambrisentan tert-butylammonium salt. These salts can be crystalline, amorphous, anhydrous or in a hydrated form.

These salts of ambrisentan are characterized by excellent stability and good solubility in water. Therefore, the new salts are more suitable candidates for preparation of pharmaceutical composition than the described forms.

The salts of ambrisentan were prepared by reaction of ambrisentan with pharmaceutical acceptable acids, as phoshoric acid, and also with pharmaceutical acceptable bases, as sodium hydroxide and *tert*.-butylamine, in organic solvents in water or in mixtures of organic solvent/water.

### Detailed description of the invention

Ambrisentan has a molecule with amphiphilic properties. It could form salts with acids so as with bases. pKa of the carboxylic group is 0.93±0.10 and pKa of nitrogen in pyrimidine ring is 2.98±0.30. For example, potassium, sodium, lithium alkylammonium salts, as well as hydrochloride, sulphate, phosphonate, hydrobromide, alkyl or arylsulphonates, could be prepared.

The salts of ambrisentan with pharmaceutical acceptable acids or bases have properties suitable for formulation (preparation of final drug forms) due to their better solubility in water or in buffers and excellent chemical stability.

The salts of ambrisentan of the present invention were prepared from ambrisentan reacting with pharmaceutically acceptable acids such as phoshoric acid, and also with pharmaceutically acceptable bases such as sodium hydroxide or *tert*-butylamine, in organic solvents, in water or in a mixture solvent/water. These salts of ambrisentan are characterized by excellent stability and good solubility in water. Therefore, the new salts are more suitable candidates for preparation of pharmaceutical composition than the described forms.

The invention relates mainly to ambrisentan phosphate salt, ambrisentan sodium salt and ambrisentan tert-butylammonium salt. These salts can be crystalline, amorphous, anhydrous or in hydrated forms.

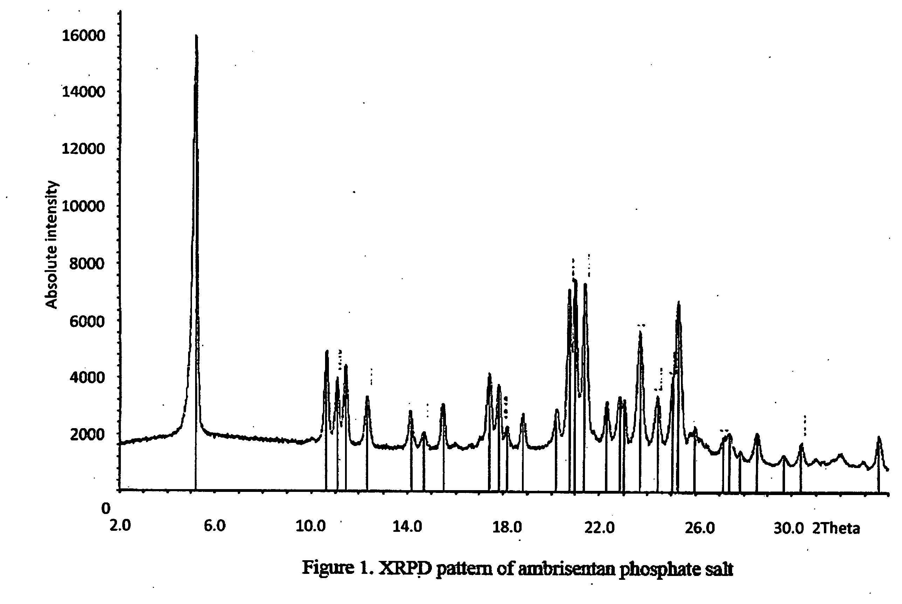

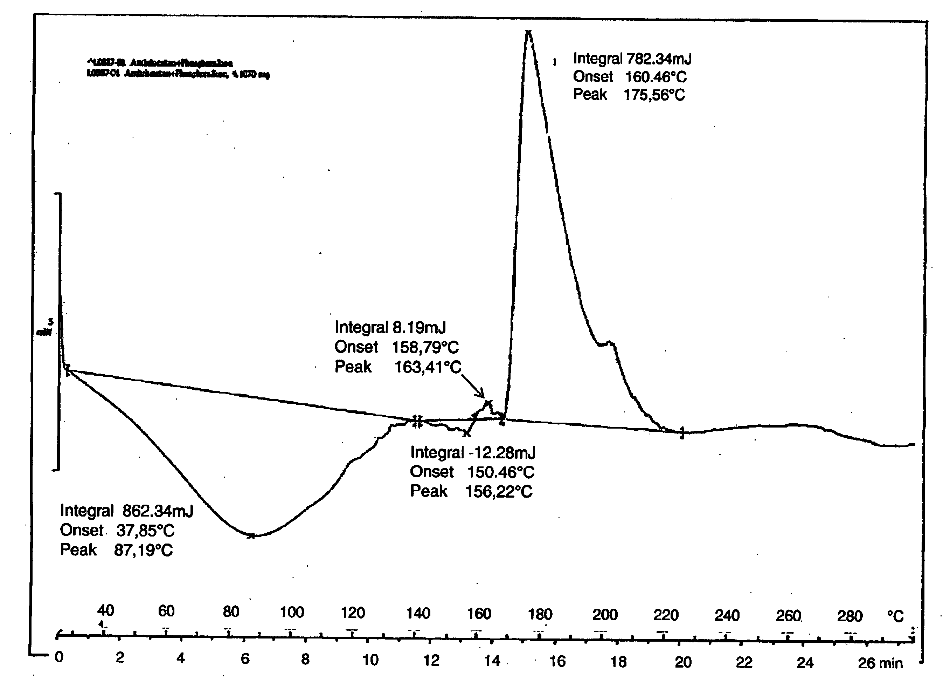

The new prepared ambrisentan phosphate salt can be characterized by X-ray powder diffraction pattern (Figure 1). Characteristic reflections of crystalline ambrisentan phosphate salt were observed at the following 2-theta values of 5.2, 10.7, 12.4, 15.5, 17.4, 21.4, 23.7 and 25.26 ± 0.2°; the main reflections were observed at 5.2, 10.7, 17.4 and 21.4 ± 0.2° degree 2-theta. The DSC-thermogram of ambrisentan phosphate salt is depicted in Figure 2.

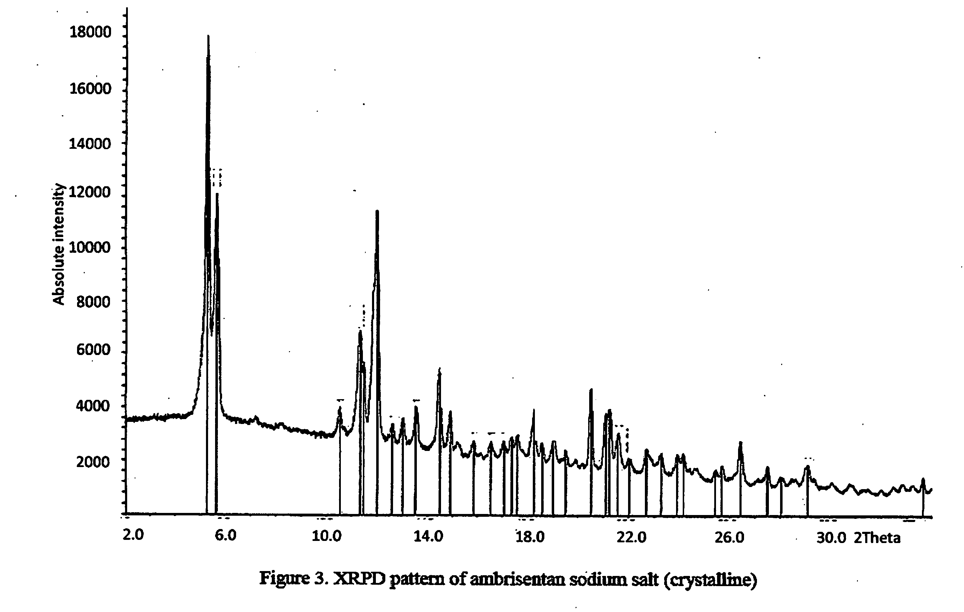

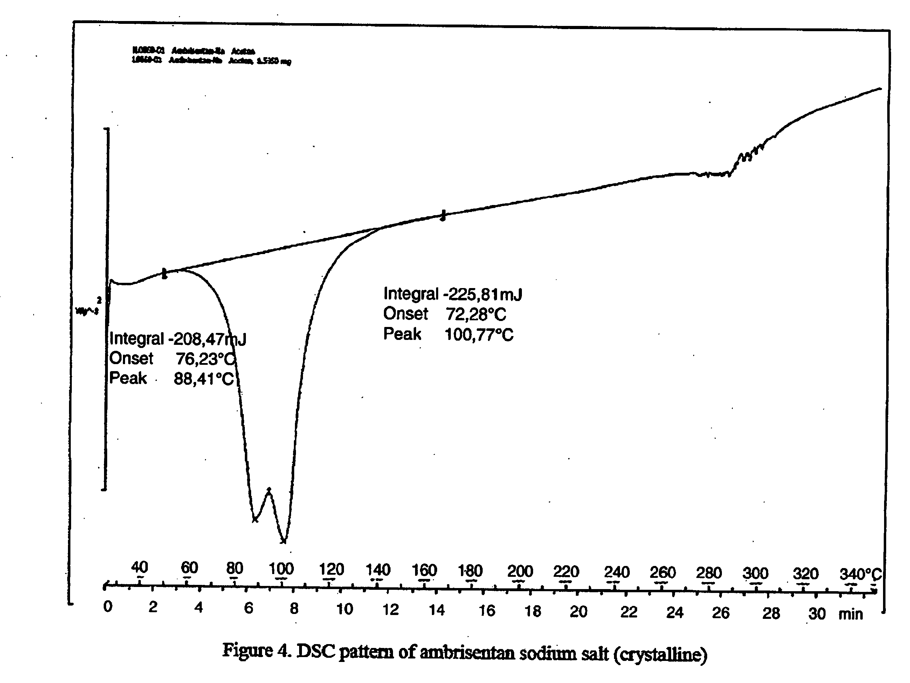

The crystalline ambrisentan sodium salt can be characterized by X-ray powder diffraction pattern (Figure 3). Characteristic reflections of crystalline ambrisentan phosphate salt were observed at the following 2-theta values of 5.3, 5.6, 12.0, 14.5, 18.3, 20.5 and 26.4 ± 0.2°; the main reflections were observed at 5.3, 12.0, 14.5 and 20.5 ± 0.2° degree 2-theta. The DSC-thermogram of crystalline ambrisentan sodium salt is depicted in Figure 4.

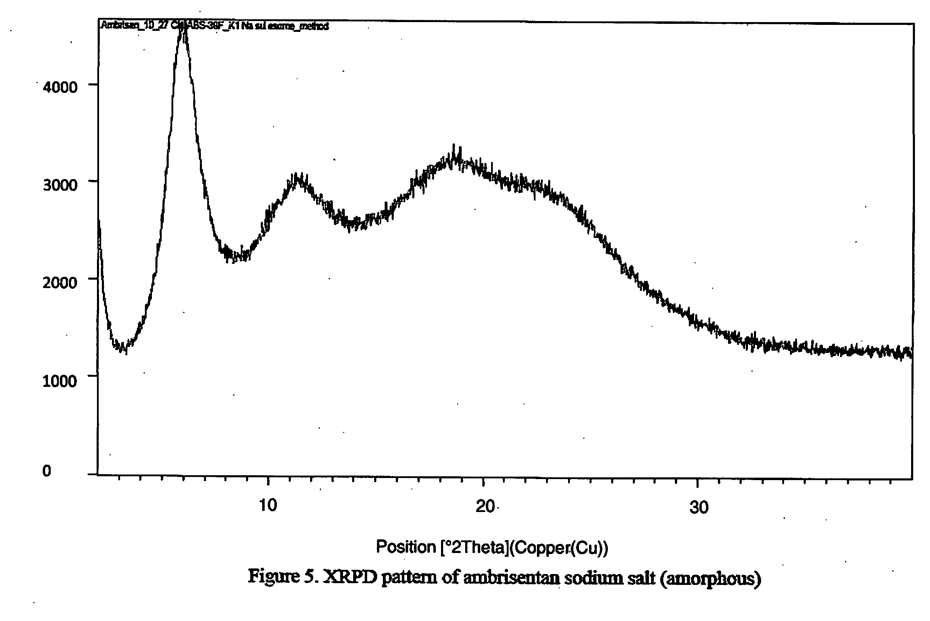

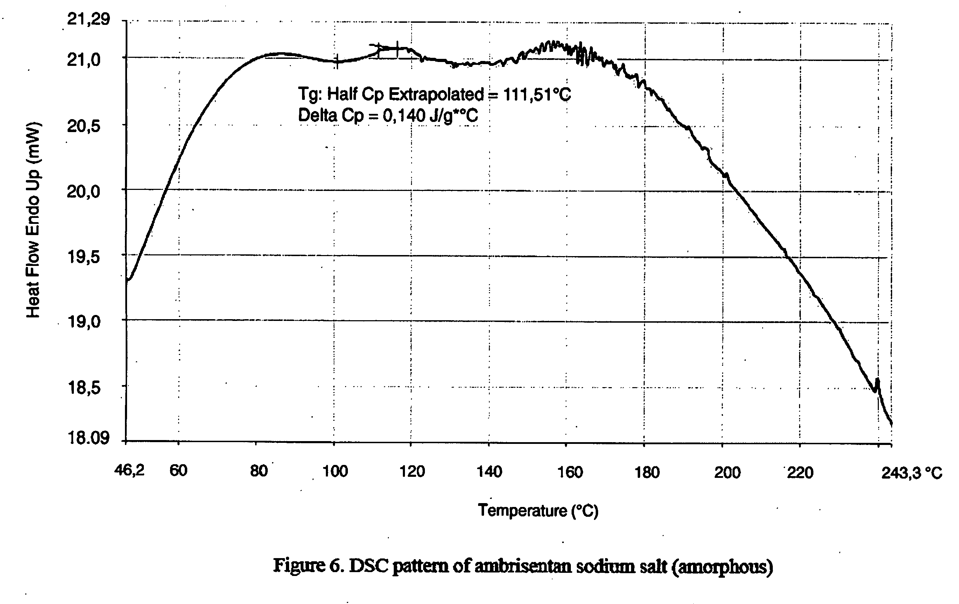

The amorphous ambrisentan sodium salt can be characterized by X-ray powder diffraction pattern depicted in Figure 5. The DSC-thermogram of amorphous ambrisentan sodium salt is depicted in Figure 6.

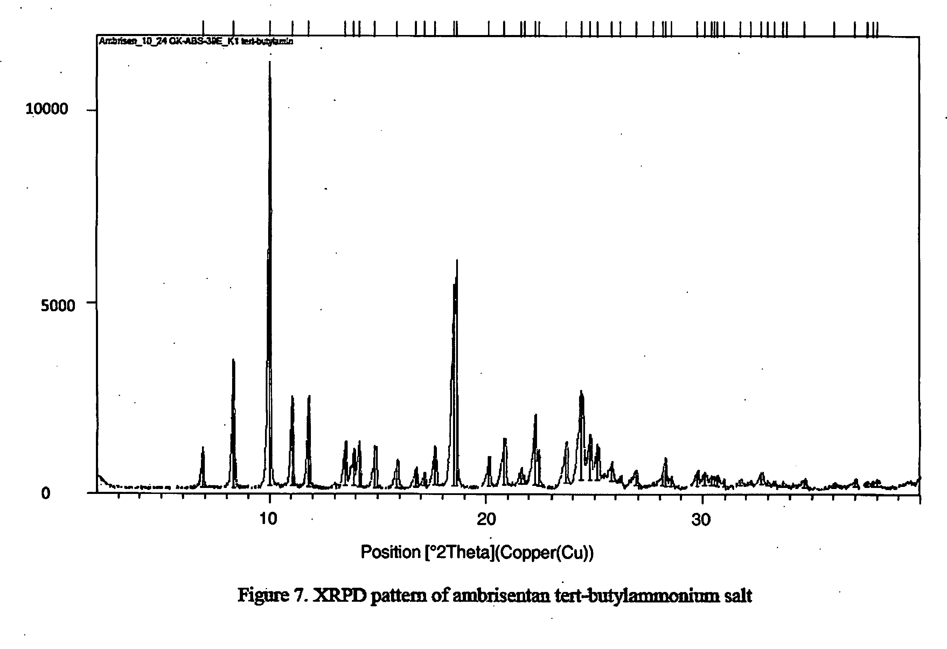

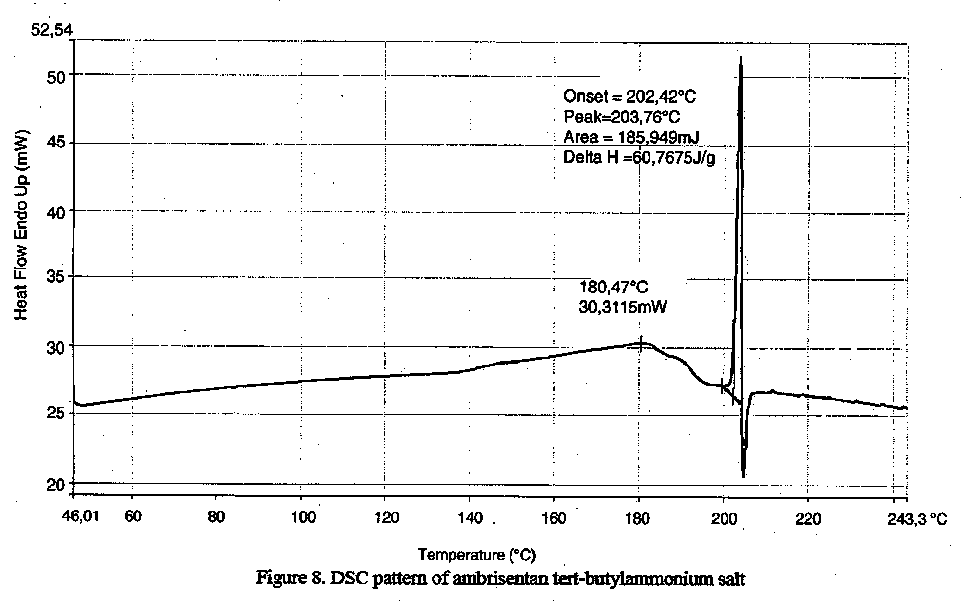

The ambrisentan tert-butylammonium salt can be characterized by X-ray powder diffraction pattern (Figure 7). Characteristic reflections of crystalline ambrisentan tert-butylammonium salt were observed at the following 2-theta values of 8.3, 10.0, 11.1, 11.8, 14.9, 18.6, 20.9, 22.3 and 24.4 ± 0.2°; the main reflections were observed at 8.3, 10.0, 11.8, 14.9, 18.6 and 24.4 ± 0.2° degree 2-theta. The DSC-thermogram of ambrisentan tert-butylammonium salt is depicted in Figure 8.

Approximate solubility values of the described ambrisentan salts are summarised in Table 1. Solubility of all the salts are considerably higher compared to solubility of ambrisentan in the salt free form.

**Table. 1 Solubility of ambrisentan solid forms in water at 25 °C**

| | Solubility (mg/ml) |
|---|---|
| Ambrisentan (Form R) | ∼ 0.05 |
| Ambrisentan phosphate salt | ∼ 0.1 |
| Ambrisentan sodium salt (crystalline) | >5 |
| Ambrisentan sodium salt (amorphous) | >10 |
| Ambrisentan tert-butylammonium salt | >6 |

Samples of all the salts were stored at 25 °C at 65% relative humidity for 1 month. All the samples adsorbed a minimal amount of water and could be considered as compounds with low hygroscopicity. All these salts are suitable for use in pharmaceutical formulations, in particular regarding to storage stability.

The ambrisentan phosphate salt can be prepared by mixing of ambrisentan solution with phosphoric acid in amount in range of 0,9 to 1,1 equivalents and evaporation of the solvent. The ambrisentan sodium salt can be prepared by mixing of an ambrisentan solution with sodium hydroxide or sodium alkoxide, e.g. sodiumm methoxide or ethoxide, in an amount in the range of 0,9 to 1,1 equivalents and evaporation of the solvent. The ambrisentan tert-butylammonium salt was prepared by mixing of an ambrisentan solution with tert-butylamine in an amount in the range of 0,9 to 1,1 equivalents and evaporation of the solvent.

The described salts can be crystallized from various organic solvents, in particular from water, C₁ to C₅ ketones (e.g., acetone, 2-butanone, 3-methyl-2-butanone, 2-pentanone, 3-pentanone), C₁ to C₃ alcohols (e.g., methanol, 2-propanol), C₁ to C₃ alkyl esters of acetic acid (e.g., ethyl acetate), acetonitrile, tetrahydrofuran, or from mixtures of these solvents. The preferred crystallization of the salts is from a mixture of an alcohol or a ketone and water at a temperature in the range of-18 °C to the boiling point of the solvent.

The ambrisentan phosphate salt is preferably prepared adding of 1 equivalent of phosphoric acid in methanol. The crystalisation of the phosphate salt is carried out at a low temperature in the range od -10 to -20 °C.

The ambrisentan sodium salt was prepared on two forms, crystalline and amorphous. The amorphous salt is preferably obtained reacting 1 equivalent of an aqueous solution of NaOH or a methanolic solution of sodium methoxide, with an alcoholic solution, preferably methanolic solution, of free ambrisentan at room temperature. The solution is evaporated to dryness or the solution is lyophilized (freeze dried) or spray-dried.

The obtained amorphous sodium salt is crystallized from ketone, in particular acetone and crystalline sodium salt is gained.

The ambrisentan tert-butylammonium salt was prepared by mixing of an ambrisentan solution, in particular ethanolic solution, with tert-butylamine at the temperature in the range of 20 to 25 °C.

### X-ray powder diffraction (XRPD)

X-ray powder diffraction measurements were performed with a Stoe Stadi-P transmission diffractometer using a linear position sensitive detector and with X'PERT PRO diffractometer using X'CELERATOR detector. Cu-Kα1 radiation (λ=1.542 Å) was used. Samples were investigated in flat preparation.

### Differential scanning calorimetry (DSC)

DSC measurements (Fig. 2 and Fig. 4) were performed with a Mettler DSC822e apparatus and DSC measurements (Fig. 6 and Fig. 8) were performed with a PerkinElmer DSC Pyris1. Al-crucibles (40µL) with sealed lid and pinhole were used. Measurements were carried out in a nitrogen gas flow of 50mL/min (Mettler DSC822e) and 20ml/min (PerkinElmer DSC Pyris1) and a heating rate of 10°/min.

### Brief description of drawings

Fig. 1 depicts the XRPD pattern of ambrisentan phosphate salt.
Fig. 2 depicts the DSC pattern of ambrisentan phosphate salt.
Fig. 3 depicts the XRPD pattern of crystalline ambrisentan sodium salt.
Fig. 4 depicts the DSC pattern of crystalline ambrisentan sodium salt.
Fig. 5 depicts the XRPD pattern of amorphous ambrisentan sodium salt.
Fig. 6 depicts the DSC pattern of amorphous ambrisentan sodium salt.
Fig. 7 depicts the XRPD pattern of ambrisentan tert-butylammonium salt.
Fig. 8 depicts the DSC pattern of ambrisentan tert-butylammonium salt.

The invention is elucidated in greater detail in the following working examples. These examples are of an illustrative nature only and do not limit the scope of the invention in any way.

### EXAMPLES

### Example 1

### Preparation of ambrisentan (Form R) according to WO2010091877, Example 7

Lithium amide (30.4 g; 1.32 mol) was suspended in N,N dimethylformamide (200 ml). A solution of (2S)-2-hydroxy-3-methoxy-3,3-diphenylpropanoic acid (120.0 g; 0.44 mol) in *N,N-*dimethylformamide (750 ml) was added dropwise at 20 °C within 45 minutes, and the mixture was stirred for additional 10 minutes. A solution of 4,6-dimethyl-2 (methyl sulfonyl)pyrimidine (90.3 g; 0.48 mol) in *N,N* dimethylformamide (500 ml) was added dropwise within 20 minutes, and the mixture was stirred for 17 hours. Water (3000 ml) and 2M citric acid (800 ml) were added to the reaction mixture. The product was extracted three times with dichloromethane (2000 ml). The combined organic phases were dried over sodium sulfate and the solvent was evaporated under reduced pressure. The oily yellow residue was crystallized from a mixture of isopropanol (800 ml) and water (2000 ml). The formed crystals were filtered off, washed with water and dried (98.5 g), mp 156-158 °C.

### Example 2

### Preparation of ambrisentan phosphate salt

To a stirred solution of Ambrisentan (1 g, 2.64 mmol) in methanol (10 ml) was added phosphoric acid (0.30 g, 2.64 mmol) at room temperature. The mixture was stirred for 2 h, then the reaction mixture was concentrated in a stream of nitrogen. The concentrated solution was allowed to crystallize at -18 °C for 24 h. The phosphate salt was obtained in the form of white powder (0.85 g), mp 172-175 °C.

XRPD according to Figure 1.

DSC according to Figure 2.

### Example 3

### Preparation of ambrisentan sodium salt (crystalline)

To a stirred solution of Ambrisentan (1 g, 2.64 mmol) in methanol (10 ml) was added sodium hydroxide (0.11 g, 2.64 mmol) at room temperature. The mixture was stirred for 2 h, then the solvent was evaporated in a stream of nitrogen. The residue was then recrystallized from acetone, and the precipitated solid was filtered off, washed with acetone and dried at room temperature. The sodium salt was obtained in the form of white powder (0.72 g), mp 82-85 °C.

XRPD according to Figure 3.

DSC according to Figure 4.

### Example 4

### Preparation of ambrisentan sodium salt (amorphous)

To a stirred solution of Ambrisentan (1 g, 2.64 mmol) in methanol (10 ml) was added a 25% solution of sodium methoxide in methanol (0.57 g, 2.64 mmol) at room temperature. The mixture was stirred for 2 h, then the solvent was evaporated in a stream of nitrogen. The salt was obtained in the form of white powder (1.02 g), Tg = 111-112 °C.

XRPD according to Figure 5.

DSC according to Figure 6.

### Example 5

### Preparation of ambrisentan tert-butylammonium salt

To a stirred solution of Ambrisentan (1 g, 2.64 mmol) in ethanol (10 ml) was added tert-butylamine (0.2 g, 2.73 mmol) at room temperature. The mixture was then stirred overnight under nitrogen at room temperature, and the precipitated solid (0.68 g) was filtered off, washed with ethanol and dried at room temperature, mp 201-203 °C.

XRPD according to Figure 7.

DSC according to Figure 8.

## Claims

1. A salt of (*S*)-2-(4,6-dimethylpyrimidin-2-yloxy)-3-methoxy-3,3-diphenylpropanoic acid (ambrisentan) with phosphoric acid of formula Ia.

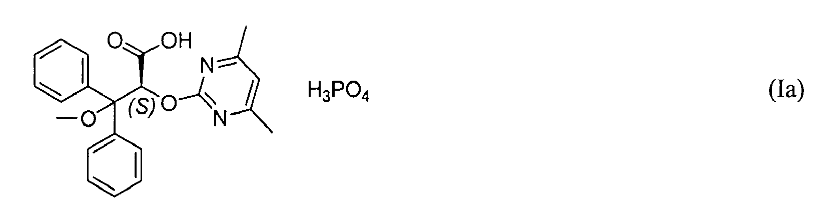

2. The salt according to claim 1, **characterized by** main peaks in its powder X-ray diffraction pattern with reflections at 5.2, 10.7, 17.4 and 21.4 ± 0.2° degree 2-theta.

3. The salt according to claims 1 or 2, **characterized by** futher peaks in its powder X-ray diffraction pattern with reflections at 12.4, 15.5, 23.7 and 25.26 ± 0.2° degree 2-theta.

4. A process for the preparation of a salt according to claims 1 or 2, which comprises reaction of ambrisentan with phosphoric acid in a solvent selected from water, C₁ to C₃ alcohols, C₁ to C₅ ketones, C₁ to C₃ esters of acetic acid, acetonitrile, tetrahydrofuran and the mixtures thereof.

5. The sodium salt of (S)-2-(4,6-dimethylpyrimidin-2-yloxy)-3-methoxy-3,3-diphenylpropanoic acid (ambrisentan) of formula Ib

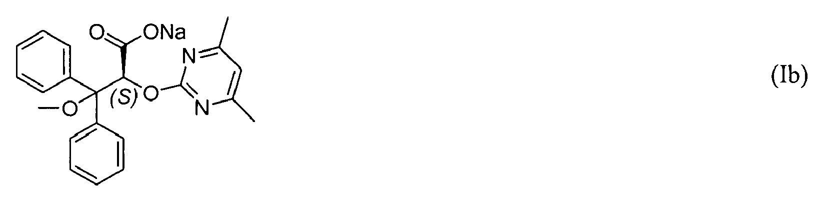

6. The salt according to claim 5 in an amorphous form.

7. The salt according to claim 5, **characterized by** main peaks in its powder X-ray diffraction pattern with reflections at 5.3, 12.0, 14.5 and 20.5 ± 0.2° degree 2-theta.

8. The salt accorging to claims 5 or 7, **characterized by** futher peaks in its powder X-ray diffraction pattern with reflections at, of 5.6, 18.3 and 26.4 ± 0.2°degree 2-theta.

9. A process for the preparation of a salt according to claims 5 or 6, which comprises reaction of ambrisentan with sodium hydroxide or sodium methoxide in a solvent selected from water, C₁ to C₃ alcohols, C₁ to C₅ ketones, C₁ to C₃ esters of acetic acid, acetonitrile, tetrahydrofuran and mixtures thereof and concentrating of the obtained solution to dryness.

10. A process for the preparation of a salt according to claims 5 or 7, which comprises crystallisation of the sodium salt of ambrisentan in a solvent selected from water, C₁ to C₃ alcohols, C₁ to C₅ ketones, C₁ to C₃ esters of acetic acid, acetonitrile, tetrahydrofuran and mixtures thereof.

11. The tert-butylammonium salt of (*S*)-2-(4,6-dimethylpyrimidin-2-yloxy)-3-methoxy-3,3-diphenylpropanoic acid (ambrisentan) of formula Ic.

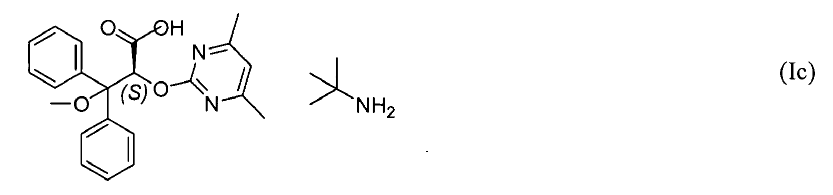

12. The salt according to claim 11, **characterized by** main peaks in its powder X-ray diffraction pattern with reflections at 8.3, 10.0, 11.8, 14.9, 18.6 and 24.4 ± 0.2° degree 2-theta.

13. The salt according to claims 11 or 12, **characterized by** futher peaks in its powder X-ray diffraction pattern with reflections at 11.1, 20.9, 22.3 ± 0.2°;degree 2-theta.

14. A process for the preparation of a salt according to claims 11 or 12, which comprises reaction of ambrisentan with tert-butylamine in a solvent selected from water, C₁ to C₃ alcohols, C₁ to C₅ ketones, C₁ to C₃ esters of acetic acid, acetonitrile, tetrahydrofuran and mixtures thereof.

15. Use of salts according to claims 1, 2, 5, 6, 7, 11 or 12 for preparation of pharmaceutical compositions.
